# EUROPÄISCHE PATENTANMELDUNG

(11) **EP 0 552 430 A1**
(43) Veröffentlichungstag der Anmeldung: **28.07.1993**
(21) Anmeldenummer: 92118879.3
(22) Anmeldetag: 04.11.1992
(51) Int. Cl.: A61B 17/04, A61B 17/28, A61B 1/06

(54) **Instrument mit einem zangenartigen Nadelhalter**

(30) Priorität: 20.01.1992 DE 4201337
(71) Anmelder: Storz, Karl, Dr.med. h.c., D-78532 Tuttlingen (DE)
(72) Erfinder: Storz, Karl, Dr.med. h.c., D-78532 Tuttlingen (DE)
(74) Vertreter: Witte, Alexander, Dr.-Ing.

(57) **Zusammenfassung**

Die Erfindung betrifft ein Instrument mit einem zangenartigen Nadelhalter mit die Nadeln haltenden Maulteile, die durch Griffelemente am patientenfernen Ende des Instrumentes betätigbar sind, für endoskopische Operationen.

Um die zum Nähen in Körperhöhlen vorzunehmenden Manipulationen weitgehend automatisch ausführen zu können, ist durch die Erfindung vorgesehen, daß die Maulteile des Nadelhalters am patientennahen Ende eines Außenschaftes (14) gebogene Rohrstücke (2, 3) aufweisen, in denen die entsprechend gebogene Nadel (4) angeordnet und durch die Kraft eines während der Bewegung der Rohrstücke zu- und abschaltbaren Magneten (4) gehalten wird, wobei das andere Rohrstück (2) ständig magnetisiert ist und bei der rückwärtigen Öffnungsbewegung der Rohrstücke gegeneinander die Nadel herauszieht.

Dadurch spart der Arzt Zeit, und das Ergebnis des operativen Eingriffs wird verbessert. Ferner ist nicht erforderlich, daß der Arzt hierzu die früher benötigte hohe Geschicklichkeit haben muß.

## Beschreibung

Die Erfindung bezieht sich auf ein Instrument mit einem zangenhaltigen Nadelhalter mit die Nadel haltenden Maulteilen, die durch Griffelemente am patientenfernen Ende des Instrumentes betätigbar sind, für endoskopische Operationen.

Es sind bereits derartige Nadelbalter bekannt, die jedoch lediglich dazu dienen, Wenden außerhalb der Körperhöhle zu nähen. Dazu benötigt man zwei derartige Nadelhalter und zumeist auch zwei Personen, die beim Nähen zusammenwirken müssen.

Die Anlegung von Nähten innerhalb von Körperhöhlen stellt dagegen hohe Anforderungen an die Geschicklichkeit des Operateurs.

Es ist weiter ein zangenartiges Instrument bekannt, bei dem der Außenschaft in das eine Maulteil ausläuft, wähnrend das andere durch ein bewegliches Griffelement am patientenfernen Ende betätigbar ist (Fa. Karl Storz GmbH & Co., Tuttlingen, Nr. 27078BJ). Hierbei ist auch schon bekannt, einen mit den Griffelementen verbundenen bewegbaren Permantentmagneten über eine Verbindungsstange zu führen, durch die die Magnetkraft zu den Maulteilen geführt wird. Zum Nähen in Körperhöhlen ist dieses Instrument jedoch nicht vorgesehen und auch nicht geeignet.

Der Erfindung liegt die Aufgabe zugrunde, die nach dem Stand der Technik zum Nähen in Körperhöhlen vorzunehmenden Manipulationen weitgehend automatisch auszuführen, d. h. das Nähen mit einem einzigen Instrument durchzuführen.

Zur Lösung dieser Aufgabe sind die kennzeichnenden Merkmale des Anspruchs 1 vorgesehen.

Dadurch spart der Arzt Zeit, und das Ergebnis des operativen Eingriffs wird verbessert. Ferner ist nicht erforderlich, daß der Arzt die früher hierzu benötigte hohe Geschicklichkeit haben muß.

In weiterer Ausgestaltung der Erfindung sind die kennzeichnenden Merkmale der Unteransprüche vorgesehen.

Weitere Vorteile und Einzelheiten der Erfindung ergeben sich aus der nun folgenden Beschreibung eines Ausführungsbeispiels unter Hinweis auf die Zeichnung. In dieser zeigen:
- Fig. 1: eine Seitenansicht auf die erfindungsgemäße Ausführungsform;
- Fig. 2: eine Ansicht auf das patientenferne Ende der Ausführungsform nach der Fig. 1 und
- Fig. 3: eine Ansicht auf das paatientennahe Ende der Ausführungsform gemäß der Fig. 1 in vergrößerndem Maßstab.

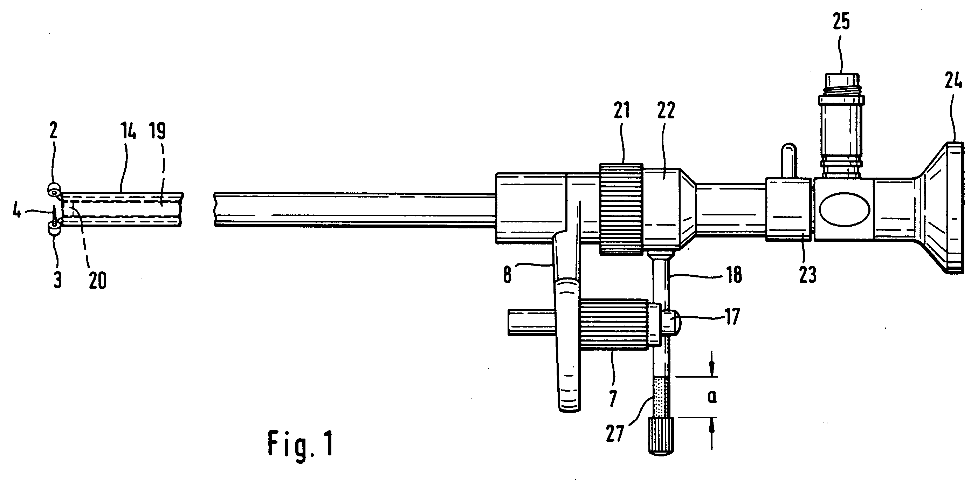

Fig. 1 zeigt links das patientennahe Ende des Instrumentes mit einem oberen Rohrstück 3 und einem unteren Rohrstück 2, wobei in dem Rohrstück 3 die Nadel 4 angeordnet ist.

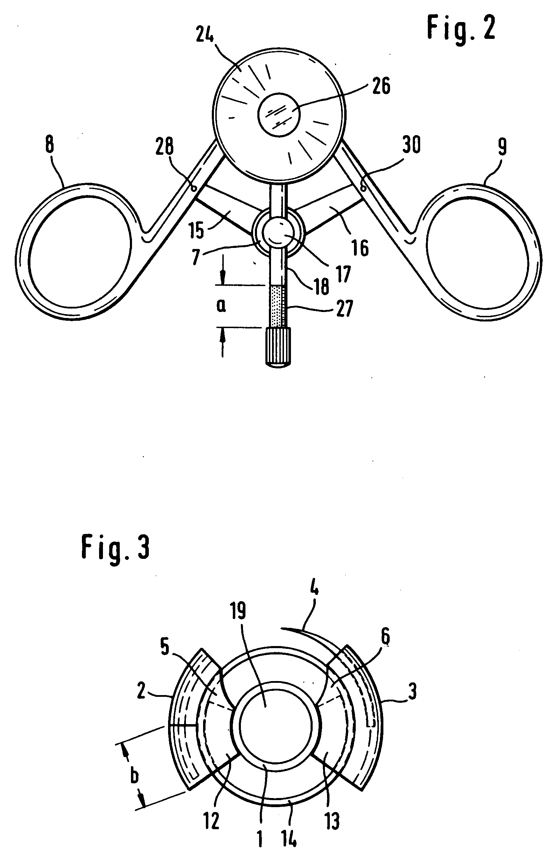

Die Einzelheiten hierfür werden später anhand der Fig. 3 erläutert werden.

In dem Außenschaft 14 ist der Innenschaft mit dem Sehrohr 19 angeordnet, an dessen patientennahem Ende 20 das Objektiv des Sehrohres 19 mit unterbrochenen Linien angedeutet ist.

Derartiges ist bei Endoskopen nach dem Stand der Technik bestens bekannt und muß deshalb nicht im einzelnen dargestellt werden.

Weiter rechts sieht man das Griffelement 8, hinter dem ein weiteres hier nicht sichtbares Griffelement 9 angeordnet ist. Diese beiden Griffelemente stehen mit dem patientennahen linken Ende in Wirkverbindung, wie dies im Prinzip bei anderen Instrumenten schon bekannt ist und deshalb hier nicht im einzelnen dargestellt werden muß. Mit der Rändelmutter 21 sind diese Teile zusammengeschraubt. Das weiter rechts sichtbare konische Teil 22 ist mit der Übertragungsstange 18 fest verbunden, die zur Übertragung der Wirkung des Permanentmagneten 7 dient, wie später noch im einzelnen erläutert wird. Die Führung 17 des Permanentmagneten 7 gleitet hierzu auf der Stange 18. Dabei zeigt die Stange 18 im untersten Bereich a eine Isolierung, so daß dann, wenn die Führung über diesen isolierten Teil bewegt wird, die Übertragung der Magnetkraft von dem Permanentmagneten 7 über die Stange 18 durch den konischen Teil 22 zu dem Rohrstück 3 am patientennahen Ende unterbrochen wird.

Weiter rechts sieht man den Bajonettverschlußring 23 herkömmlicher Art, der dazu dient, das hier nicht sichtbare Sehrohr 19 mit dem Instrument zu verriegeln. Dabei bildet das Sehrohr 19 eine Einheit mit der Augenmuschel 24, die bekanntlich zur Aufnahme des Okulars des Objektives dient und mit einem Lichtleiteranschluß 25 versehen ist.

Diese Teile mit dem Sehrohr 19 sind dem Fachmann für sich bestens bekannt und müssen deshalb ebenfalls nicht im einzelnen erläutert werden. Das gilt auch für den Bajonettverschluß mit dem Ring 23.

Fig. 2 zeigt oben wieder die schon erwähnte Augenmuschel mit dem Okular 26. Darunter sieht man die beiden Griffelemente 8 und 9, die mit den beiden beweglichen Hebeln 15 und 16 scherenartig in Verbindung stehen, die unten die erwähnte Führung 17 zeigen, hinter der der Permanentmagnet 7 sichtbar ist. Darunter erkennt man einen Bereich a mit einem Isolationsmaterial 27, das nur in diesem Bereich auf die Stange 18 aufgetragen ist.

Wenn die beiden Griffelemente 8 und 9 auseinander bewegt werden, bewegen sich die beiden Hebel 15 und 16 um ihre Gelenke 28 und 30 und bewegen dadurch den Permanentmagneten 7 nach oben. Beim Zusammendrücken der beiden Griffelemente 8 und 9 wird dagegen der Permanentmagnet 7 nach unten bewegt, bis er über den Bereich a mit dem Isolationsmaterial 27 gelangt. Dadurch wird die Übertragung der Magnetkraft durch die Stange 18 auf das Rohrstück 3 unterbrochen.

Fig. 3 zeigt links das Rohrstück 2, das der Biegung des Außenschaftes 14 angepaßt ist und über das Segment 12 mit einem der beiden Griffelemente 8, 9 in Verbindung steht. Dabei läuft das Segment 12 zu dem Rohrstück 2 über die Zunge 5 aus. Ebenso sieht man rechts einen zungenartigen Auslauf 6 des Segmentes 13 zu dem Rohrstück 3.

Das Rohrstück 2 ist in seinem der Nadel 4 abgewandten Bereich b stark magnetisiert. Die lichte Weite des Rohrstückes 2 ist ebenso wie die des Rohrstückes 3 zur Aufnahme der entsprechend gebogenen Nadel 4 vorgesehen, die teilweise in dem Rohrstück 3 sitzt und deshalb nur zu einem Teil voll sichtbar ist.

Im Innenschaft 1 ist das Sehrohr 19 angeordnet, das bereits anhand der Fig. 1 erwähnt wurde, dort jedoch nicht sichtbar ist.

Im nachfolgenden wird die Wirkungsweise des Erfindungsgegenstandes erläutert:
Wenn die beiden Griffelemente 8, 9 zusammengedrückt werden, bewegen sich die Rohrstücke 2, 3 aufeinander zu, wobei die Nadel 4 einen Nadelstich durch das zu nähende Gewebe innerhalb der Körperhöhle ausführt. Sie ist nämlich durch den starken Permanentmagneten 7 stark magnetisiert und hält deshalb die Nadel 4 mit Sicherheit fest, bis sie in die Röhre 2 um einen gewissen Betrag eingefahren ist. Erst dann gleitet der Permanentmagnet 7 über die Isolierung 27, wodurch die Magnetwirkung des Rohrstückes 3 unterbrochen und somit aufgehoben wird. Der starke Permanentmagnet im Bereich b des Rohrstückes 2 zieht nunmehr die Nadel 4 vollends in das Rohrstück 2 hinein, so daß sie zusammen mit dem nicht dargestellten Faden aus dem Gewebe des Patienten ganz herausgezogen wird.

Danach können die beiden Griffelemente 8 und 9 wieder voneinander entfernt werden, wodurch sich die beiden Rohrstücke 2 und 3 zangenartig voneinander entfernen und das Gewebe des Patienten völlig freigeben. Es kann nämlich sein, daß durch die beiden Rohrstücke 2 und 3 das Gewebe des Patienten zuvor zusammengedrückt worden ist, was erwünscht ist, um das Gewebe beim Nähen festzuhalten.

Nunmehr kann das Instrument aus der Körperhöhle herausgezogen und der Faden verknotet werden. Das Instrument kann ferner für einen weiteren Stich zum Nähen im Bedarfsfall vorbereitet werden.

Der oben erwähnte Nähvorgang kann durch die Augenmuschel 24 und das Sehrohr 19 vom Arzt beobachtet und somit unter Sichtkontrolle gesteuert werden.

Die Erfindung ist nicht auf die dargestellte Ausführungsform beschränkt. Der Fachmann hat vielmehr die Möglichkeit, Abwandlungen hiervon im Rahmen der Ansprüche auszuführen.

## Patentansprüche

1. Instrument mit einem zangenartigen Nadelhalter mit die Nadel haltenden Maulteilen, die durch Griffelemente am patientenfernen Ende des Instrumentes betätigbar sind, für endoskopische Operationen, dadurch gekennzeichnet, daß die Maulteile des Nadelhalters am patientennahen Ende eines Außenschaftes (14) gebogene Rohrstücke (2,3) aufweisen, in denen die entsprechend gebogene Nadel (4) angeordnet und durch die Kraft eines während der Bewegung der Rohrstücke zu- und abschaltbaren Magneten (7) gehalten wird, wobei das andere Rockstück (2) ständig magnetisiert ist und bei der rückwärtigen Öffnungsbewegung der Rohrstücke gegeneinander die Nadel herauszieht.

2. Instrument nach Anspruch 1, dadurch gekennzeichnet, daß die Rohrstücke (2,3) in Anpassung an den Außenschaft (14) kreisbogenförmig ausgebildet sind und mit Segmenten (12,13) in dem Außenschaft zu ihrer Betätigung in Verbindung mit den Griffelementen (8,9) stehen, wobei die Segmente zu den Rohrstücken zungenartig auslaufen.

3. Instrument nach Anspruch 2, dadurch gekennzeichnet, daß die Griffelemente (8,9) mit dem Permanentmagneten (7) über eine starre Verbindungsstange (18) in Verbindung stehen, über die eine Führung (17) gleitbar ist, die in einem Teilbereich (a) mit einer isolierenden Beschichtung versehen ist, durch die die Verbindung der Magnetwirkung zu dem Rohrstück (3) unterbrochen wird.

4. Instrument nach Anspruch 1, dadurch gekennzeichnet, daß das ständig magnetische Rohrstück (2) in seinem der Nadel (4) abgewandten Bereich (b) aus einem permanent magnetischem Werkstoff mit starker Magnetkraft besteht.

5. Instrument nach Anspruch 1, dadurch gekennzeichnet, daß der Außenschaft (14) einen Innenschaft (1) zur Aufnahme eines Sehrohres (19) aufweist.
